(19) Europäisches Patentamt

European Patent Office

Office européen des brevets

(11) **EP 4 245 213 A1**

(12) **EUROPEAN PATENT APPLICATION**

(43) Date of publication:
**20.09.2023 Bulletin 2023/38**

(21) Application number: **22192062.2**

(22) Date of filing: **25.08.2022**

(51) International Patent Classification (IPC):
**A61B 5/022** (2006.01) **A61B 5/024** (2006.01)
**A61B 5/00** (2006.01)

(52) Cooperative Patent Classification (CPC):
**A61B 5/022; A61B 5/02416; A61B 5/6824;
A61B 5/7264;** A61B 2560/0223

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.03.2022 US 202263321095 P
25.05.2022 US 202217752911**

(71) Applicant: **Microlife Corporation
Taipei City 114 (TW)**

(72) Inventors:
• **Wang, Ching-Fu
114 Taipei City (TW)**
• **Li, Shih-Zhang
114 Taipei City (TW)**
• **Chen, You-Yin
114 Taipei City (TW)**
• **Lin, Chia-Ming
114 Taipei City (TW)**

(74) Representative: **Appleyard Lees IP LLP
15 Clare Road
Halifax HX1 2HY (GB)**

(54) **SYSTEM FOR ESTIMATING BLOOD PRESSURES USING PHOTOPLETHYSMOGRAPHY SIGNAL ANALYSIS**

(57) A system for estimating BPs using a PPG signal analysis comprises an upper-arm wearable apparatus (11), a cuff-based BP measuring apparatus (12), a PPG signal receiver and analyzer (13), and a PPG to BP estimator and calibrator (14). The upper-arm wearable apparatus (11) senses modeling-used PPG waveform signals. The cuff-based BP measuring apparatus (12) obtains real PVR waveforms and real BPs. The PPG signal receiver and analyzer (13) is configured to process the modeling-used PPG waveform signals and derive modeling-used characteristic parameters, and have modeling-used personal information parameters. The PPG to BP estimator and calibrator (14) is configured to calculate estimated BPs based on the modeling-based characteristic parameters and the modeling-used personal information parameters, store a calibration model which approximately fits relationship between the estimated BPs and the real BPs; and calculate modeling-used calibrated-estimated BPs using the calibration model.

FIG. 1

EP 4 245 213 A1

**Description**

**CROSS-REFERENCE TO RELATED INVENTION**

This patent application claims the benefits of United States Provisional Application No. 63/321,095 filed March 17, 2022 and United States non-Provisional Application No. 17/752,911 filed May 25, 2022, and the disclosures are incorporated herein by reference in its entirety.

**BACKGROUND OF THE INVENTION**

1. Field of the Invention

The present invention relates to a system for estimating blood pressures (BPs) using photoplethysmography (PPG) signal analysis, in particular to a system for estimating blood pressure by processing PPG waveform signals detected at the upper arm of a subject and using machine learning algorithms to further have accurately estimated BPs and a heart rate.

2. Description of Related Art

Hypertension is a major cardiovascular risk factor contributing to various medical conditions, diseases, and events such as heart attacks, heart failure, aneurisms, strokes, and kidney disease. Thus, it is a critical cardiovascular parameter for early identification of cardiovascular diseases (CVDs). There is a well-established need for blood pressure and other vital sign monitoring, whether such monitoring occurs in a hospital setting, a physician's office, a patient's home or office, and whether such monitoring occurs while the individual is at rest or engaged in an activity, such as sitting, walking, exercising, or sleeping.

A continuous measurement of BPs is necessary for medical monitoring and diagnosis by physicians. Traditional 24-h BP measuring apparatuses can monitor BPs with a cuff at regular intervals through repeated inflation and deflation day and night. Such measurements at night cause insomnia or interrupt sleeping in health people, and are uncomfortable, discontinuous, and unsuitable for daily use.

Recently, wearable healthcare devices have been demonstrated to be successful for personal health monitoring over the long term without troublesome life interference and to help professionals understand how a patient's multiple chronic conditions interact. To calculate BPs using a wrist-type device, many studies attempted to estimate BPs using PPG signals on the wrist-type device for simpler design. Usually, patients with hypertension also have other individual differences, which results in interference of PPG waveforms and decreases the fitting accuracy of the Gaussian processes (GP) regression model used in a conventional estimating system. These individual differences can be affected by several factors, environmental conditions, and/or experimental errors during the data measurement. Because cardiovascular features and signals vary among conditions and change with time, determining how much the BP estimation varies depending on factors or in terms of time is important.

Jia-Wei Chen et al. accordingly proposed a paper entitled "A Data-Driven Model with Feedback Calibration Embedded Blood Pressure Estimator Using Reflective Photoplethysmography" (Sensors 22.5 (2022): 1873), the teachings of which are incorporated herein by reference in their entirety. In this study, the BP estimation is performed based on multi-age-grouping models by PPG morphology characteristic parameters and personal information parameters as features. The translation of PPG signals into arterial blood pressure (ABP) is described in another non-patent literature entitled "PPG2ABP: Translating Photoplethysmogram (PPG) Signals to Arterial Blood Pressure (ABP) Waveforms using Fully Convolutional Neural Networks" (written by Nabil Ibtehaz, M. Sohel Rahman; Electrical Engineering and Systems Science: Signal Processing, published on May 5, 2020), the teachings of which are incorporated herein by reference in their entirety. However, the PPG signals of the both foregoing studies are obtained from the wrist-type device or finger-type device.

A PPG sensor included in the wrist-type device generally comprises an LED light source and a photodetector. The LED emits light to the skin tissue and the photodetector keeps track of how much light is reflected, i.e., the degree of absorption. The amount of reflected light originally from the PPG sensor is proportional to the volume of blood flowing in the illuminated skin area. On the other hand, if the PPG signals are detected from an upper arm which is quite closer to the heart than a wrist or a finger, such arm-based signals are superior in the well presentation of cardiovascular features and the BP estimation. However, different signal processing approaches were required for upper-arm and wrist signals, indicating that wrist-based BP estimation models should not be generalized directly to arm PPG signals. Furthermore, if the estimation of central artic blood pressures (CBPs) can be further made based on the arm PPG signals, it is more useful to predict the occurrence of hypertension and related cardiovascular events than the BP estimation.

# EP 4 245 213 A1

## SUMMARY OF THE INVENTION

In view of the deficiency of the current technology, the present application provides a method for estimating blood pressures (BPs) using a photoplethysmography (PPG) signal analysis and a system using the same. The method or system is comfortable, continuous, and suitable for all-day use, and the accuracy of estimation of BPs and/or central artic blood pressures (CBPs) are sufficiently improved. In addition, the PPG analysis and measurement may be integrated into an upper-arm wearable apparatus or a brachial cuff pulse volume plethysmography (PVP) apparatus to accurately estimate the CBPs of a user.

In view of the foregoing aspect, in one embodiment, the present application provides a method for calibrating and estimating BPs using a PPG signal analysis comprising the steps of:

> providing an upper-arm wearable apparatus adapted to sense modeling-used PPG waveform signals from a plurality of subjects wearing the upper-arm wearable apparatus;
> processing the modeling-used PPG waveform signals and deriving modeling-used characteristic parameters from the modeling-used PPG waveform signals;
> having modeling-used personal information parameters from the plurality of subjects;
> calculating estimated BPs based on the modeling-used characteristic parameters and the modeling-used personal information parameters by dividing at least one of the modeling-used personal information parameters into a plurality of groups;
> providing a cuff-based BP measuring apparatus to obtain pulse volume recording (PVR) waveforms and real BPs of the plurality of subjects;
> establishing a calibration model to approximately fit relationship between the estimated BPs and the real BPs;
> obtaining user's estimated BPs for a user wearing the upper-arm wearable apparatus based on user's characteristic parameters and user's personal information parameters; and
> inputting the user's estimated BPs and real BPs to the calibration model to have calibrated-estimated BPs and a heart rate for the user.

In view of the foregoing aspect, in one embodiment, the present application provides a method for estimating CBPs using a PPG signal analysis comprising the steps of:

> providing an upper-arm wearable apparatus adapted to sense modeling-used PPG waveform signals from a plurality of subjects wearing the upper-arm wearable apparatus;
> processing the modeling-used PPG waveform signals and deriving modeling-based characteristic parameters from the modeling-used PPG waveform signals;
> having modeling-used personal information parameters from the plurality of subjects;
> calculating estimated BPs based on the modeling-used characteristic parameters and the modeling-used personal information parameters by dividing at least one of the modeling-used personal information parameters into a plurality of groups;
> providing a cuff-based BP measuring apparatus to obtain real pulse volume recording (PVR) waveforms and real BPs of the plurality of subjects;
> establishing a calibration model to approximately fit relationship between the estimated BPs and the real BPs;
> calculating modeling-used calibrated-estimated BPs from the estimated BPs and the real BPs using the calibration model;
> establishing a prediction model by processing modeling-used PPG waveform signals using an Approximation Network and a Refinement Network to have modeling-used refined PVR waveforms based on the real PVR waveforms;
> establishing a linear regression equation to fit correlation between waveform parameters of the modeling-used refined PVR waveforms and the modeling-used calibrated-estimated BPs from the plurality of subjects;
> obtaining user's calibrated BPs for a user wearing the upper-arm wearable apparatus based on user's characteristic parameters and user's personal information parameters;
> inputting the user's estimated BPs and real BPs to the calibration model to have user's calibrated-estimated BPs and a heart rate for the user;
> obtaining a user's refined PVR waveform from a user's PPG waveform signal using the prediction model; and
> substituting the user's calibrated-estimated BPs, the heart rate and waveform parameters of the user's refined PVR waveform into the linear regression equation to have estimated CBPs.

In view of the foregoing aspect, in one embodiment, the present application provides a system for estimating BPs using a PPG signal analysis comprising:

3

an upper-arm wearable apparatus including a PPG sensor and sensing modeling-used PPG waveform signals from a plurality of subjects wearing the upper-arm wearable apparatus; and
a cuff-based BP measuring apparatus obtaining real PVR waveforms and real BPs of the plurality of subjects;
a PPG signal receiver and analyzer configured to:

process the modeling PPG waveform signals and derive modeling-used characteristic parameters from the modeling-used PPG waveform signals; and
have modeling-used personal information parameters from the plurality of subjects;

a PPG to BP estimator and calibrator configured to:

calculate estimated BPs based on the modeling-used characteristic parameters and the modeling-used personal information parameters by dividing at least one of the modeling-used personal information parameters into a plurality of groups;
store a calibration model which approximately fits relationship between the estimated BPs and the real BPs; and calculate modeling-used calibrated-estimated BPs from the estimated BPs and the real BPs using the calibration model to have calibrated-estimated BPs.

In view of the foregoing aspect, in one embodiment, the present application provides a system for estimating CBPs using a PPG signal analysis comprising:

an upper-arm wearable apparatus including a PPG sensor and sensing modeling-used PPG waveform signals from a plurality of subjects wearing the upper-arm wearable apparatus; and
a cuff-based BP measuring apparatus obtaining real PVR waveforms and real BPs of the plurality of subjects;
a PPG signal receiver and analyzer configured to:

process the modeling PPG waveform signals and derive modeling-used characteristic parameters from the modeling-used PPG waveform signals; and
have modeling-used personal information parameters from the plurality of subjects;

a PPG to BP estimator and calibrator configured to:

calculate estimated BPs based on the modeling-used characteristic parameters and the modeling-used personal information parameters by dividing at least one of the modeling-used personal information parameters into a plurality of groups;
store a calibration model which approximately fits relationship between the estimated BPs and the real BPs; and calculate modeling-used calibrated-estimated BPs from the estimated BPs and the real BPs using the calibration model to have calibrated-estimated BPs;

a PPG to PVR transformer configured to:
store a prediction model which processes modeling-based PPG waveform signals using an Approximation Network and a Refinement Network to have modeling-used refined PVR waveforms based on the real PVR waveforms; and
a CBP estimator configured to:

store a linear regression equation which fits correlation between waveform parameters of the modeling-used refined PVR waveforms and the modeling-used calibrated-estimated BPs from the plurality of subjects; and substitute calibrated-estimated BPs, a heart rate and waveform parameters of a refined PVR waveform derived from a user into the linear regression equation to have estimated CBPs.

## BRIEF DESCRIPTION OF THE DRAWINGS

In order to sufficiently understand the essence, advantages and the preferred embodiments of the present invention, the following detailed description will be more clearly understood by referring to the accompanying drawings.

FIG. 1 is a block diagram showing the BP and/or CBP estimating system and its subsystems according to an embodiment of the present application;
FIG. 2A is a schematic diagram showing an upper-arm wearable apparatus having an embedded PPG sensor according to an embodiment of the present application;

FIG. 2B is circuit diagram showing a PPG analog preprocessing circuit according to an embodiment of the present application;

FIG. 3 is a schematic waveform diagram showing a processed PPG waveform including characteristic parameters according to an embodiment of the present application;

FIG. 4A and 4B are statistical diagram showing relationship between real SBP and calibrated-estimated SBP in the analysis of correlation coefficient according to an embodiment of the present application;

FIG. 5A-5D shows asynchronous upper-arm PPG and PVR waveform signals under the trimming and aligning processing according to an embodiment of the present application;

FIG. 6A shows an upper-arm PPG waveform according to an embodiment of the present application;

FIG. 6B shows a preprocessed PPG waveform according to an embodiment of the present application;

FIG. 6C shows an approximate PVP waveform according to an embodiment of the present application;

FIG. 6D shows a refined PVP waveform according to an embodiment of the present application;

FIG. 7A shows a PPG waveform signal derived from an upper-arm wearable apparatus according to an embodiment of the present application;

FIG. 7B shows a refined PVR waveform after the deep learning processing according to an embodiment of the present application;

FIG. 7C shows a real PVR waveform derived from a cuff-based BP measuring apparatus according to an embodiment of the present application;

FIG. 7D shows the refined PVR waveform and real PVR waveform both pieced together according to an embodiment of the present application.

## DETAILED DESCRIPTION OF THE INVENTION

The following description shows the preferred embodiments of the present invention. The present invention is described below by referring to the embodiments and the figures. Thus, the present invention is not intended to be limited to the embodiments shown, but is to be accorded the principles disclosed herein. Furthermore, that various modifications or changes in light thereof will be suggested to persons skilled in the art and are to be included within the spirit and purview of this application and scope of the appended claims.

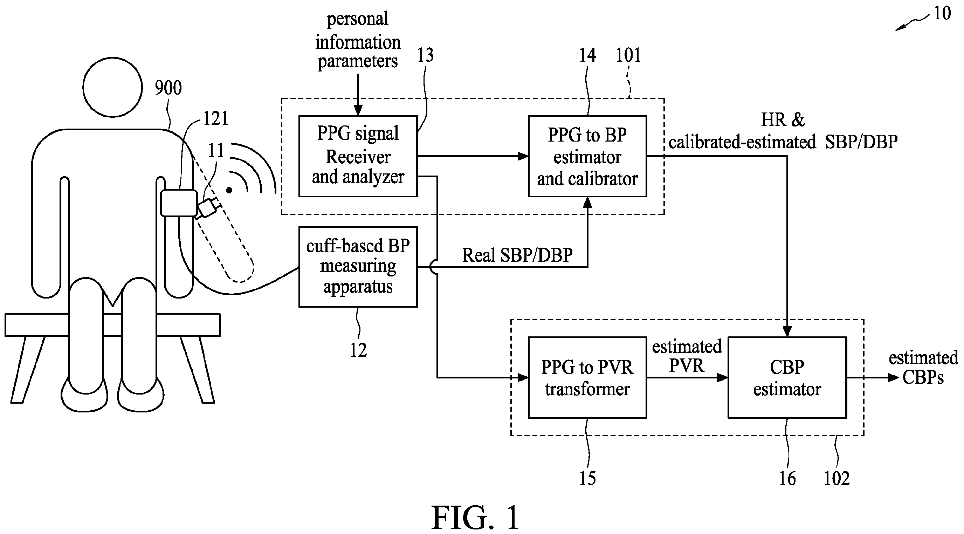

FIG. 1 is a block diagram showing a BP and/or CBP estimating system 10according to an embodiment of the present application. In general, a subject 900 statically sit on a chair for steady-state measurement, but measurements also can be performed every interval based on the stable subject scenario during daytime and nighttime for clinical use of 24-h ABPM. The subject 900 among a plurality of subjects wears an upper-arm wearable apparatus 11 or a cuff 121 included in a cuff-based BP measuring apparatus 12 respectively during different periods. In the other embodiment, the cuff-based BP measuring apparatus 12 at least includes a pump, a relief valve and a pressure sensor. The cuff-based BP measuring apparatus 12 has been calibrated so as to provide the PPG to BP estimator and calibrator 14 with an accurate blood pressure. The upper-arm wearable apparatus 11 is integrated with a PPG sensor (not shown) designed to measure PPG waveform signals and/or an optical biosensor capable of sensing other vital signs from a right or left upper arm only. The PPG waveform signals and/or other vital signs are wirelessly transmitted to and shown in a PPG signal receiver and analyzer 13 such as a notebook, computer or smart phone. Also, the PPG signal receiver and analyzer 13 may let the upper-arm wearable apparatus 11 be at an auto-monitoring mode to perform measurements every an interval (e.g. 30 min) based on the stable subject scenario during daytime and nighttime for the clinical use of 24-h ambulatory blood pressure monitoring (ABPM).

The characteristic parameters are extracted from the PPG waveform signals for modeling use or clinic use by the PPG signal receiver and analyzer 13. That is, the feature extraction is performed on the PPG waveform signals to have the modeling-used (i.e. especially for model-established use) characteristic parameters in this embodiment. Furthermore, the personal information parameters such as age and gender are inputted to the PPG signal receiver and analyzer 13. In other embodiment, the PPG to BP subsystem 101 further includes an input unit for providing the PPG signal receiver and analyzer 13 with the personal information parameters. Afterward, a PPG to BP estimator and calibrator 14 receives the characteristic parameters and personal information parameters from the PPG signal receiver and analyzer 13. Furthermore, it uses the age grouping method and further calibrates preliminary values by machine learning (ML) algorithms or other algorithms (e.g. regression algorithms, artificial neural networks (ANN), fuzzy logic, and support vector machine) to have more accurately estimated SBP (systolic BP), estimated DBP (diastolic BP), and HR (heart rate), as will be discussed in detail below. In view of above, the PPG to BP subsystem 101 including the PPG signal receiver and analyzer 13 and the PPG to BP estimator and calibrator 14 may be a computer or a circuit system, or its partial or all functions may be performed at or integrated into the upper-arm wearable apparatus 11, and outputs the calibrated-estimated SBP, calibrated-estimated DBP, and HR. In other embodiment, the PPG to BP subsystem 101 further includes a display unit (not shown) for displaying the calibrated-estimated SBP, calibrated-estimated DBP, and HR to the users.

In this embodiment, the BP and/or CBP estimating system 10 further includes a PPG to CBP subsystem 102. The PPG to CBP subsystem 102 comprises a PPG to PVR transformer 15 and a CBP estimator 16. The PPG to PVR transformer 15 converts the PPG waveform signals to refined PVR waveforms using deep machine learning, as will be further discussed below. The CBP estimator 16 further uses linear regression equations to estimate CBPs in clinic use. The linear regression equations are initially established as the method taught by U.S. Patent No. 201502725112, the teachings of which are incorporated herein by reference in their entirety, to fit correlation between waveform parameters of the approximated PVR waveforms, estimated BPs, and real CBPs measured from the plurality of subjects. In view of above, the PPG to CBP subsystem 102 may be a computer or a circuit system, or its partial or all functions may be performed at or integrated into the upper-arm wearable apparatus 11.

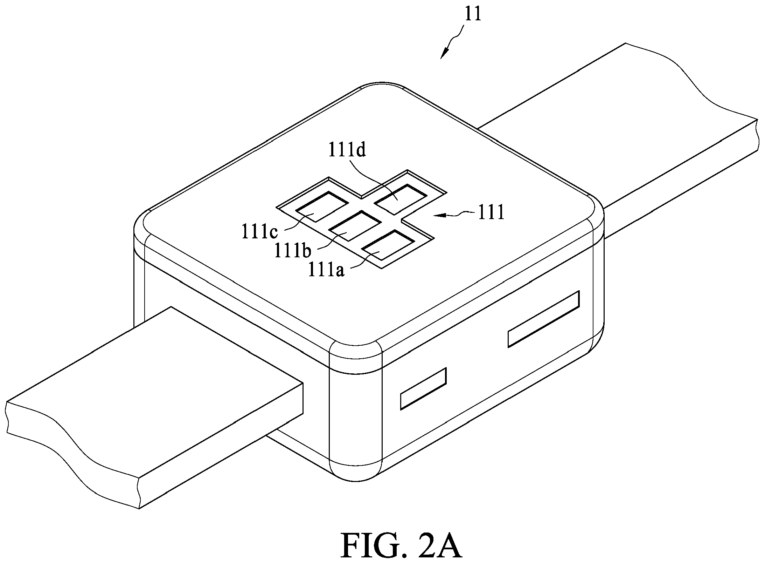

FIG. 2A is a schematic diagram showing the upper-arm wearable apparatus 11 having a PPG sensor 111 embedded in its back. The PPG sensor 111 is composed of a multi-wavelength light source with three individual green, red, or infrared light-emitting diodes (LEDs) (111a, 111c, 111d) and a photodiode (PD) I11b. There is a black partition plate between the infrared light-emitting diodes (111a, 111c, 111d) and photodiode 111b, which could efficiently eliminate interference and crosstalk from external light. In this embodiment, only the green light LED is turned on for the primary estimation of BPs, the green light is transmitted by an LED into the skin, and the amount of reflective or unabsorbed light is measured using a PD, which shows the blood volume changes in the microvascular bed of the tissue.

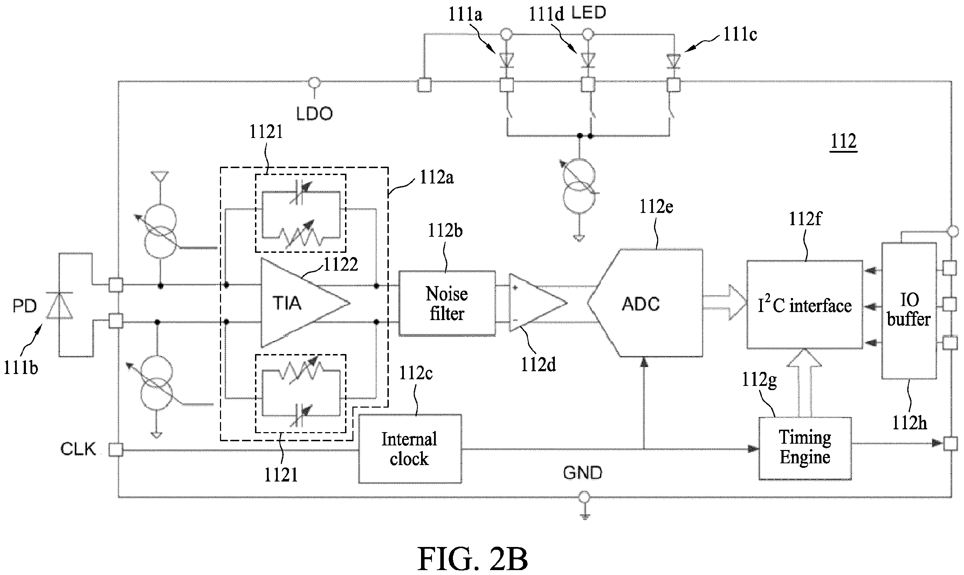

Referring to FIG. 2B, a PPG analog preprocessing circuit 112 comprises LEDs (111a, 111c, 111d), a photodiode 111b, an LED controller (not shown), a DC drift compensation circuit 112a, a noise filter 112b, an internal clock unit 112c, an amplifier 112d, an analogous-to-digital convertor (ADC) 112e, an $I^2C$ interference, a timing engine 112g, and an IO buffer 112h. The amplifier 112d is used to enhance the signal from the PD 111b, and the signal is converted to digital data by the ADC 112e. The back-scattered or reflected PPG waveform signal detected from the PD 111b is first amplified (gain = 66 dB) and low-pass filtered (filter bandwidth = 50 Hz) in the analog prepressing. The analog prepressing utilizes the drift compensation circuit 112a which comprises a trans-impedance amplifier (TIA)1122 and R-C filters 1121 to compensate or cancel the DC drift of the signal. After the PPG analog preprocessing is well done, a clean PPG waveform signal is obtained and used for the feature extraction.

The upper-arm wearable apparatus 11 may be further equipped with a G-sensor (gravity sensor) and/or a vibration device (vibration motor). When the upper-arm wearable apparatus 11 starts a measurement, the vibration device will vibrate as a reminder. Or, when the G-sensor senses an apparent motion of the upper-arm (any movement of the body will affect measurement results), it also vibrate as a reminder. In another embodiment, the upper-arm motion can be recognized from a PPG waveform signals. Moreover, the reminder device can be replaced by a buzzer.

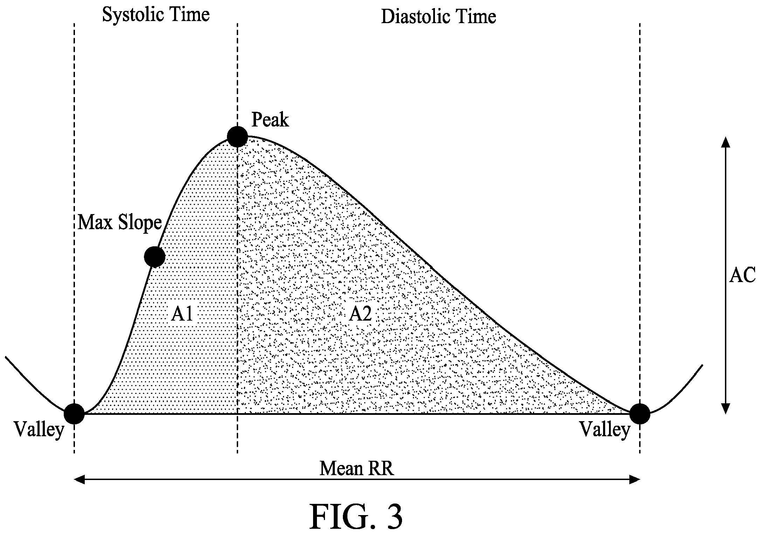

FIG. 3 is a schematic waveform diagram showing a processed PPG waveform including the characteristic parameters including waveform parameters and time-related parameters extracted by the PPG signal receiver and analyzer 13. The waveform parameters include systolic area over total area (i.e. A1/(A1+A2)), diastolic area over total area (i.e. A2/(A1+A2)), systolic area over pulse amplitude (i.e. AI/AC), diastolic area over pulse amplitude (i.e. A2/AC), and maximal amplitude over time as maximal slope, and the time-related parameters include systolic time, diastolic time and mean peak to peak interval.

The variation of the characteristic parameters is significantly different between males and females and different age groups. In order to reduce the variation, the PPG to BP prediction model performed by the PPG to BP estimator and calibrator 14 is divided in multiple groups, and they are constructed for subject-specific relation between PPG and BP. The multiple groups are classified by an age grouping method and trained using an exponential GPR (Gaussian Process Regression) algorithm. In this embodiment, 435 subjects participate in the establishment of the PPG to BP prediction model. A PPG database is divided into training dataset (306 subjects or participants as reference database) and test dataset (129 subjects or participants as validation database). For a DBP prediction model in this embodiment, the PPG database is separated by gender and age by groups of 15 years (i.e., Age < 30, $30 \leq$ Age < 45, $45 \leq$ Age < 60, $60 \leq$ Age < 75, $75 \leq$ Age). Above all, there are 10 groups trained as different models for SBP. On the other hand, the SBP prediction model is by groups of 30 years with 6 groups (i.e., Age < 30, $30 \leq$ Age < 60, $60 \leq$ Age). A plurality of variables (such as ten or nine variables) including characteristic parameters and personal information parameters are the model input parameters.

The training set was expressed as Equation (1) in the following way:

$$\{x_i, y_i\}_{i=1}^{n} \tag{1}$$

where $n$ represents the number of data sets, $x$ represents the training parameters array including characteristic parameters and personal information parameters, and $y$ represents the target value as actual BP value. A learning function $f(x_i)$ was used for transforming the input array $x_i$ into the target value $y_i$ given a model as Equation (2):

$$y_i = f(x_i) + \varepsilon_i \qquad (2)$$

where $\varepsilon_i$ represents Gaussian noise with zero mean and $\sigma_n^2$ represented the variance. As a result, the observed targets can also be described by a Gaussian distribution as Equation (3):

$$y \sim N(0, K(x,x) + \sigma_n^2 I) \qquad (3)$$

where x represents the vector of all input points $x_i$ and $K(x, x)$ represents the covariance matrix computed using a given covariance function. The covariance function could be defined by various kernel functions and could be parameterized in terms of the kernel parameters in vector $\theta$. Hence, it is possible to express the covariance function as $K(x, x|\theta)$. This model uses the exponential kernel function with a separate length scale for each predictor. The covariance function is defined as follows:

$$k(x_i, x_j|\theta) = \sigma^2_f \, exp\left[-\frac{\sqrt{(x_i - x_j)^T (x_i - x_j)}}{\sigma_l}\right] \qquad (4)$$

The kernel parameters are based on the signal standard deviation $\sigma_f$ and the characteristic length scale $\sigma_l$. The unconstrained parameterization $\theta$ is:

$$\theta_1 = log\,\sigma_l, \theta_2 = log\,\sigma_f \qquad (5)$$

Therefore, the joint distribution of the observed target values and predicted value $f(x_i)$ for a query point $i$ was given in Equation (6):

$$\begin{bmatrix} y \\ f(x_i) \end{bmatrix} \sim N(0, \begin{bmatrix} K(x,x) + \sigma_n^2 I & k(x, x_i) \\ k(x_i, x) & k(x_i, x_i) \end{bmatrix}) \qquad (6)$$

The predicted mean value $\overline{f(x_i)}$ and the corresponding variance $V(x_i)$ could be represented in Equations (7) and (8) as follows:

$$\overline{f(x_i)} = k(x, x_i)^T (K(x,x) + \sigma_n^2 I)^{-1} y \qquad (7)$$

$$V(x_i) = k(x_i, x_i) - k(x, x_i)^T (K(x,x) + \sigma_n^2 I)^{-1} k(x, x_i) \qquad (8)$$

The GPR model is a type of ML method for statistically analyzing data. The purpose is to understand the relationship between two or more variables and establish a mathematical model to predict the variables of interest. More specifically, using regression analysis, the relation function can be found and the long-term trend of BP can be estimated from the given characteristic parameters.

When a user (i.e., 25 years old) starts an PPG to BP estimation or prediction using the upper-arm wearable apparatus 11 and the PPG to BP subsystem 101 without calibration, all the age groups of trained-based models with the actual gender (i.e., male) are used to predict many BP values. The minimal mean error between the estimated SBP and real SBP from the cuff-based BP measuring apparatus 12 is calculated and the corresponded optimal age group (i.e., 30 ≤ Age < 45; but not the true age group above) is selected with calibration. Finally, the optimal age group for this user is used for the further BP estimation accurately. Furthermore, an interface based on the iOS, Android app or Windows system is designed as the easily keying in the personal information parameters and activating calibrated function by with Bluetooth Low Energy (BLE) in the upper-arm wearable apparatus 11. The chosen model was more suitable for the user's PPG characteristic parameters, and a compensated value could be calculated in variable $K(x_i, x_i)$ and BPs could be estimated using Equation (7) initially. Then, the parameters could be included in the model for personal calibration, and the optimized model and group would predict the BPs more accurately.

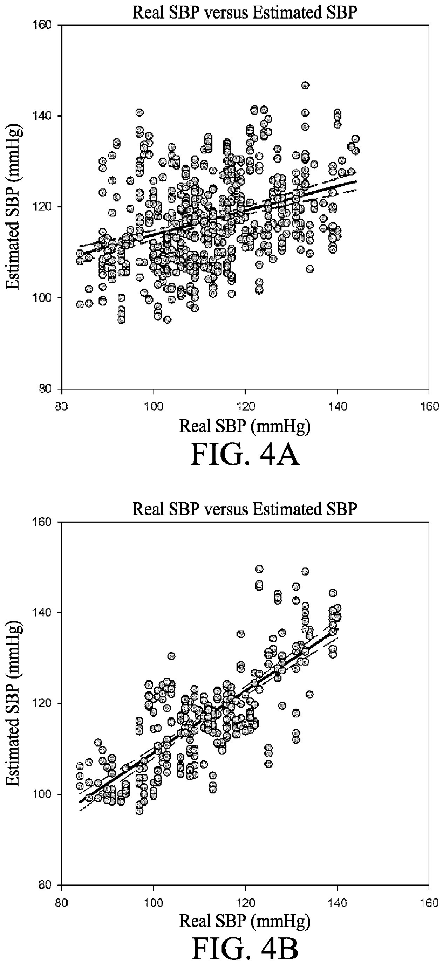

FIG. 4A and 4B are statistical diagram showing relationship between real SBP and estimated SBP in the analysis of correlation coefficient. In FIG. 4A, the PPG to SBP estimation without calibration shows a moderate correlation between the real SBP values and estimated SBP values. By contrast, the PPG to SBP estimation with calibration shows an excellent correlation between the real SBP values and calibrated-estimated SBP values as shown in FIG. 4B.

The accuracy of the PPG to BP estimation can meet the standard ANSI/AAMI/ISO 81060-2:2013. Apparently, the grades under the BHS Grading criteria get apparently better after calibration. The established and verified results are shown in Table 1 below.

Table 1

| PPG to BP Model | Without Calibration | | | | With Calibration | | | |
|---|---|---|---|---|---|---|---|---|
| Total (mmHg) | ≤5 | ≤10 | ≤15 | ΔBP | ≤5 | ≤10 | ≤15 | ΔBP |
| DBP | 40 (C) | 78.33 (B) | 91 (B) | -3.0 ± 8.11 | 50.67 (B) | 82.33 (B) | 97.33 (A) | -3.6 ± 6.33 |
| SBP | 34.33 (D) | 59.67 (D) | 80.33 (D) | -2.96 ± 0.89 | 42.67 (C) | 72.33 (C) | 83.67 (D) | -5.5 ± 8.41 |
| Interval (mmHg) | ≤15 | ≤20 | ≤30 | ΔBP | ≤15 | ≤20 | ≤30 | ΔBP |
| DBP < 60 | 79.49% | 89.74% | 100% | -11.95±5.31 | 84.62% | 97.44% | 100% | -11.42±4.24 |
| 60 ≤ DBP < 80 | 93.72% | 99.52% | 100% | -4.09±6.09 | 99.03% | 99.52% | 100% | -4.12±4.78 |
| 80 ≤ DBP | 88.89% | 100% | 100% | 7.34±5.83 | 100% | 100% | 100% | 4.05±4.29 |
| SBP < 90 | 25% | 50% | 100% | -18.54±5.27 | 33.33% | 58.33% | 100% | -17.53±4.25 |
| 90 ≤ SBP < 130 | 84.52% | 95.24% | 100% | -4.32±9.26 | 84.92% | 92.86% | 100% | -5.70±7.97 |
| 130 ≤ SBP | 69.44% | 83.33% | 100% | 11.73±8.37 | 91.67% | 100% | 100% | -0.09±7.92 |

BHS Grading criteria (mmHg, cumulative percentage): Grade A (≤5, 60%; ≤10, 85%; ≤15, 95%), Grade B (≤5, 50%; ≤10, 75%; ≤15,

90%), Grade C (≤5, 40%; ≤10, 65%; ≤15, 85%), Grade D (worse than Grade C).

ANSI/AAMI/ISO 81060-2:2013: ΔBP < 5-mmHg, Mean standard deviation < 8-mmHg.

Referring to the foregoing non-patent literature written by Nabil Ibtehaz et al., the finger-PPG database was trained by the PPG to ABP model as a pre-trained model for ABP estimation. In one embodiment, a fine-tuned model is proposed and trained based on the upper-arm PPG database from 50 subjects. The training process steps follow the method taught by Ibtehaz et al. The asynchronous databases between upper-arm PPG waveform signals (hereinafter referred to as "upper-arm PPG") and PVR waveform signals are needed to be solved in the pre-processing step, but such problem is not seen and discussed in the Ibtehaz's study. In the pre-processing step, the adjustment for the same peak number alignment and dynamic time warping method is implemented to synchronize the database between upper-arm PPG and PVR waveform signals. Also, the normalization value (Maxima and minima) for upper-arm PPG and PVR waveform signals were calculated from the upper-arm PPG database in this embodiment.

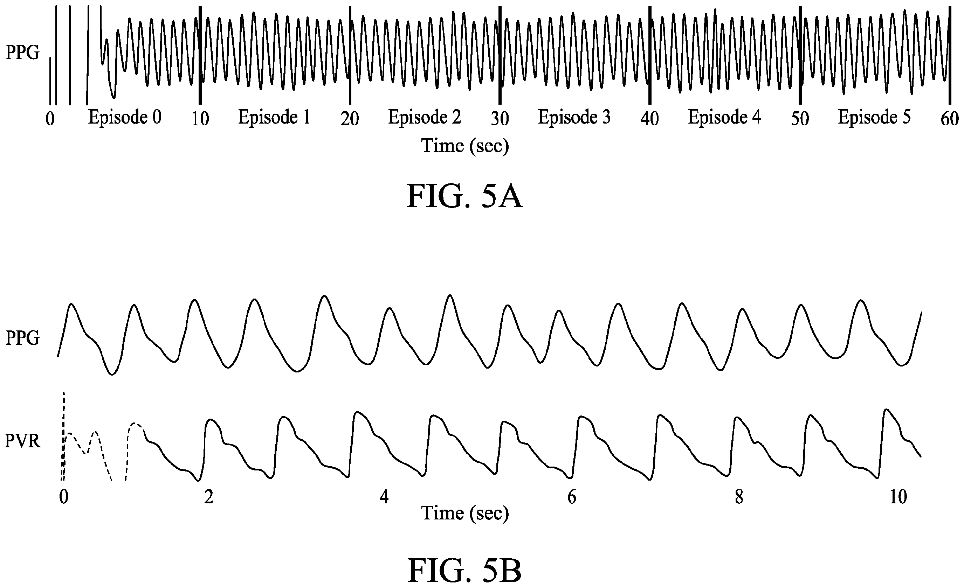

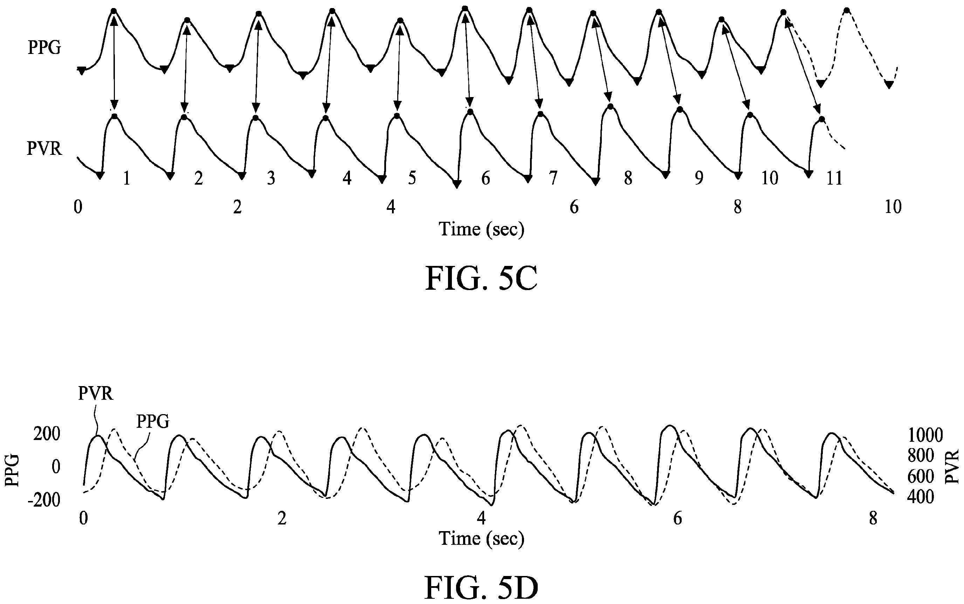

FIG. 5A-5D shows asynchronous upper-arm PPG and PVR waveform signals under the trimming and aligning processing according to an embodiment of the present application. First, the PPG waveform is split into six episodes, each with 10s. The initial episode 0 within first 10s need to be deleted because of signal distortion, and the divided PPG waveform within episodes 1-5 are further processed by averaging waveform. Similarly, the PVR waveform within the first second is also trimmed as shown in FIG. 5B. Afterward, the upper-arm PPG and PVR waveforms are synchronized with each other using the same peak number alignment and dynamic time warping method. That is, the occurrences of two peaks labeled with the same number of the upper-arm PPG and PVR waveforms are about close to each other along a time axis, as shown in FIG. 5C. Final, in FIG. 5D, two processed upper-arm PPG and PVR waveforms are pieced together.

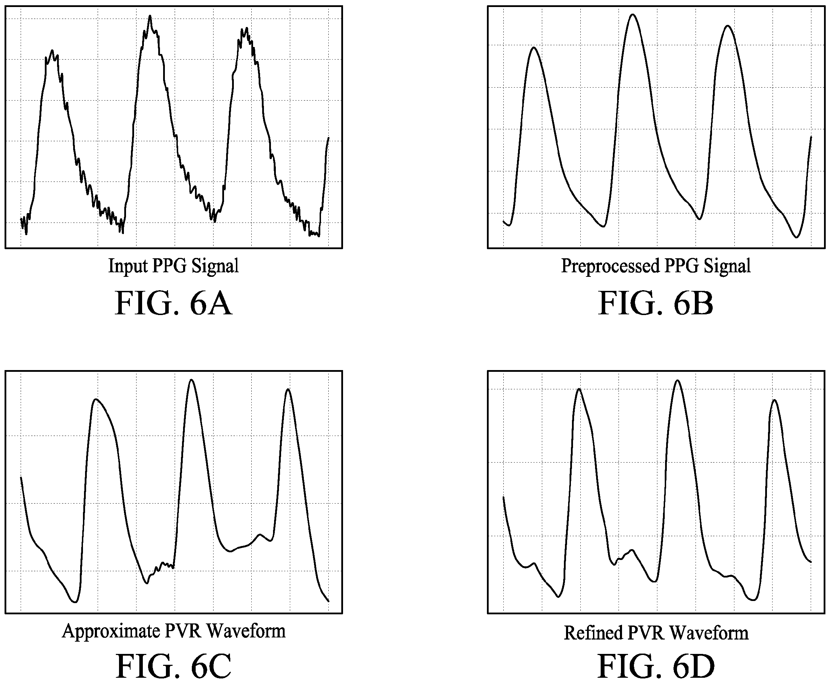

Furthermore, the steps of PPG to PVR transformation are further discussed hereinafter. The PPG2PVR algorithm proposed by Ibtehaz et al. is executed to take an upper-arm PPG waveform of 8.912 seconds long from a PPG waveform signal, as shown in FIG. 6A. Referring to FIG. 6B, the preprocessed or filtered PPG waveform is then performed with some minimal preprocessing operations to attenuate its irregularities and noises. Next, the preprocessed PPG waveform is further processed using an Approximation Network that approximates the shape of the PVR waveform based on the input upper-arm PPG waveform signal, as shown in FIG. 6C. The preliminary approximate estimated PVR waveform is further refined through a Refinement Network so as to have a refined PVR waveform in FIG. 6D. Both networks mentioned above were based one-dimensional deep supervised U-Net model architectures (U-Net model for Approximation Network and MultiResUNet model for Refinement Network).

U-Net comprises a network constructed using only convolutional layers to perform the task of semantic segmentation. The network structure is constructed using a symmetric pair of Encoder Network and Decoder Network. The Encoder Network extracts spatial features from the input, which are utilized by the Decoder Network to produce the segmentation map. The most innovative idea behind U-Net is the use of skip connections, that preserve the spatial feature maps, likely to have lost during pooling operation. Though the original U-Net is designed to perform semantic segmentation on images, for our purpose, we employ it to perform regression based on one-dimensional signals. Therefore, the two-dimensional convolution, pooling and up-sampling operations are replaced by their one-dimensional counterparts.

Moreover, all the convolutional layers other than the final one use the ReLU (Rectied Linear Unit) activation function and are batch normalized. To produce a regression output, the final convolutional layer uses a linear activation function. Moreover, deep supervision is used in the U-Net network. Deep supervision is a technique proven to reduce overall error by directing the learning process of the hidden layers. In the deeply supervised ID U-Net, an intermediate output is computed, which was a subsampled version of the actual output signal, prior to every up-sampling operation. A loss function is computed with gradually declining weight factor as the learning process goes deeper into the model. This additional auxiliary loses is used to drive the training of the hidden layers and makes the final output much superior.

The MultiResUNet model is an improved version of the U-Net model. The primary distinction between the two is the inclusion of MultiRes blocks and Res paths. Multires blocks involve a compact form of multresolutional analysis using factorized convolutions. On the other hand, Res paths impose additional convolutional operations along the shortcut connections to reduce the disparity between the feature maps of the corresponding levels of Encoder and Decoder networks. Similar to the Approximation Network, this network also consists of one-dimensional versions of convolution, pooling and up-sampling operations. The activation functions are identical as well, i.e., ReLU for all the layers but the final one, which uses a linear activation instead. The layers are also batch normalized but not deeply supervised.

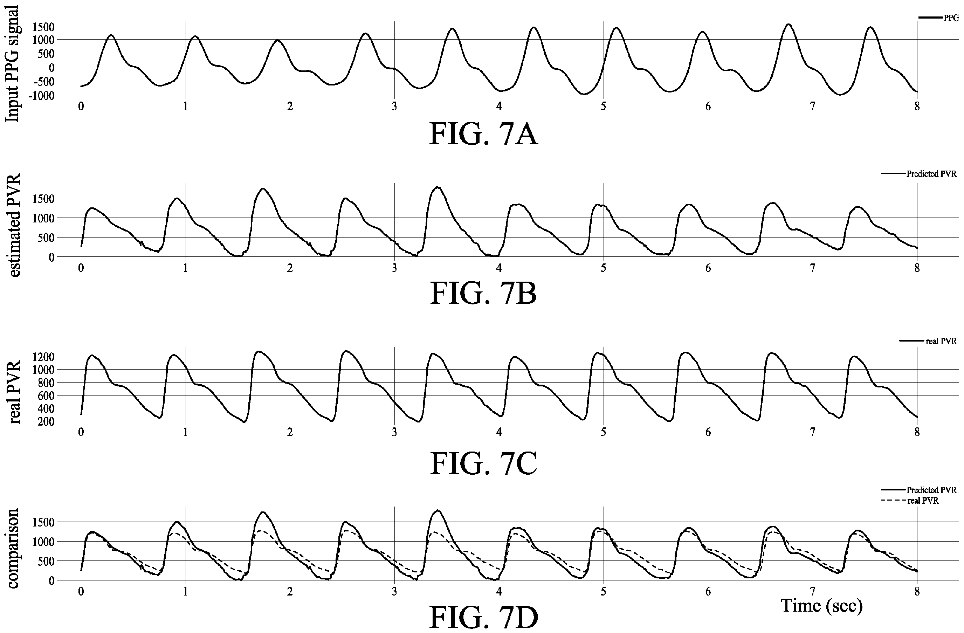

FIG. 7A shows a PPG waveform signal derived from the upper-arm wearable apparatus 11. FIG. 7B shows a refined (estimated, not real) PVR waveform after the deep learning processing of Approximation Network and Refinement Network. FIG. 7C shows a real PVR waveform derived from the cuff-based BP measuring apparatus 12. The refined (estimated) PVR waveform and real PVR waveform are both pieced together as show in FIG. 7D. The estimated PVR waveform is quite similar to the real PVR waveform.

In FIG. 1, the CBP estimator 16 further uses linear regression equations to estimate CBPs in clinic use, and substitutes the calibrated-estimated BPs and waveform parameters of the user's refined PVR waveform into the linear regression equation to have estimated CBPs. The linear regression equation taught by U.S. Patent No. 20150272512 has a central blood pressure as a dependent variable and has a pressure of the late systolic shoulder produced by wave reflections, an end-systolic pressure, an area under the waveform during systole, an area under the waveform during diastole, a pressure at end-diastole, and a heart rate as the control variables.

The linear regression equation is illustrated below:

$$SBP\text{-}C = 0.30 \times SBP2 + 0.20 \times ESP + 1.97 \times As + 0.87 \times Ad - 0.75 \times DBP$$

$$+ 1.00 \times Heart\ Rate - 58.16 \qquad (9)$$

$$PP\text{-}C = 0.26 \times SBP2 - 0.06 \times ESP + 2.61 \times As + 1.37 \times Ad - 1.73 \times DBP + 1.62 \times$$

$$Heart\ Rate - 114.64 \qquad (10)$$

wherein SBP-C represents a systolic blood pressure, PP-C represents a pulse pressure, SBP2 represents a pressure value of the late systolic shoulder produced by wave reflections, ESP represents an end-systolic pressure value, As represents an area under the waveform during systole, Ad represents an area under the waveform during diastole, DBP represents a pressure value at end-diastole, and Heart Rate represents a heart rate.

In the equations (9) and (10), SBP-C represents a systolic blood pressure and PP-C represents a pulse pressure. The regression coefficient (being constant) before each control variable and constants (-58.16, -114.64) are just exemplary. The coefficients and constants can be varied according to various estimation devices or electronic components used in the devices, but the present invention is not limited to the example.

The foregoing embodiments of the present invention have been presented for the purpose of illustration. Although the invention has been described by certain preceding examples, it is not to be construed as being limited by them. They are not intended to be exhaustive, or to limit the scope of the invention. Modifications, improvements and variations within the scope of the invention are possible in light of this disclosure. For example, the processing or calibration of waveform signals can be changed in sequence. Moreover, another function block can be added to or inserted into the function block diagram of the central blood pressure estimation device, but the added function block such as a filter and a screen showing estimated values may not affect the technical concept of the present invention.

**Claims**

1. A system for estimating BPs using a PPG signal analysis, **characterized by** comprising:

   an upper-arm wearable apparatus (11) including a PPG sensor and sensing modeling-used PPG waveform signals from a plurality of subjects (900) wearing the upper-arm wearable apparatus (11); and
   a cuff-based BP measuring apparatus (12) obtaining real PVR waveforms and real BPs of the plurality of subjects (900);
   a PPG signal receiver and analyzer (13) configured to:

   process the modeling-used PPG waveform signals and derive modeling-used characteristic parameters from the modeling-used PPG waveform signals; and
   have modeling-used personal information parameters from the plurality of subjects; and

   a PPG to BP estimator and calibrator (14) configured to:

   calculate estimated BPs based on the modeling-based characteristic parameters and the modeling-used personal information parameters by dividing at least one of the modeling-used personal information parameters into a plurality of groups;
   store a calibration model which approximately fits relationship between the estimated BPs and the real BPs; and
   calculate modeling-used calibrated-estimated BPs from the estimated BPs and the real BPs using the calibration model to have calibrated-estimated BPs.

2. The system for estimating BPs using a PPG signal analysis according to claim 1, wherein the upper-arm wearable apparatus (11) further includes a gravity sensor sensing a motion of the upper-arm of the subject (900) when the subject (900) wears the upper-arm wearable apparatus (11).

3. The system for estimating BPs using a PPG signal analysis according to claim 2, wherein the upper-arm wearable apparatus (11) further includes a reminder device which alerts the subject when the gravity sensor senses the motion.

4. The system for estimating BPs using a PPG signal analysis according to claim 1, wherein the PPG signal receiver and analyzer (13) and the PPG to BP estimator and calibrator (14) are integrated into the upper-arm wearable apparatus (11).

5. The system for estimating BPs using a PPG signal analysis according to claim 1, wherein the upper-arm wearable apparatus (11) wirelessly or by wire transmits the PPG waveform signals to the PPG signal receiver and analyzer (13).

6. The system for estimating BPs using a PPG signal analysis according to claim 1, wherein the PPG signal receiver and analyzer (13) calibrates the estimated BPs through machine learning (ML) algorithms using an age and gender grouping method.

7. A system (10) for estimating CBPs using a PPG signal analysis, **characterized by** comprising:

an upper-arm wearable apparatus (11) including a PPG sensor and sensing modeling-used PPG waveform signals from a plurality of subjects (900) wearing the upper-arm wearable apparatus (11); and
a cuff-based BP measuring apparatus (12) obtaining real PVR waveforms and real BPs of the plurality of subjects (900);
a PPG signal receiver and analyzer (13) configured to:

process the modeling-used PPG waveform signals and derive modeling-used characteristic parameters from the modeling-used PPG waveform signals; and
have modeling-used personal information parameters from the plurality of subjects; and

a PPG to BP estimator and calibrator (14) configured to:

calculate estimated BPs based on the modeling-based characteristic parameters and the modeling-used personal information parameters by dividing at least one of the modeling-used personal information parameters into a plurality of groups;
store a calibration model which approximately fits relationship between the estimated BPs and the real BPs; and
calculate modeling-used calibrated-estimated BPs from the estimated BPs and the real BPs using the calibration model;

a PPG to PVR transformer (15) configured to:
store a prediction model which processes modeling-based PPG waveform signals using an Approximation Network and a Refinement Network to have modeling-used refined PVR waveforms based on the real PVR waveforms; and
a CBP estimator (16) configured to:

store a linear regression equation which fits correlation between waveform parameters of the modeling-used refined PVR waveforms and the modeling-used calibrated-estimated BPs from the plurality of subjects (900); and
substitute calibrated-estimated BPs, a heart rate and waveform parameters of a refined PVR waveform derived from a user into the linear regression equation to have estimated CBPs.

8. The system (10) for estimating CBPs using a PPG signal analysis according to claim 7, wherein the characteristic parameters are derived from the modeling-used PPG waveform signals by performing feature extraction.

9. The system (10) for estimating CBPs using a PPG signal analysis according to claim 7, wherein the modeling-used PPG waveform signals is split into a plurality of episodes each with an identical interval, an initial episode is deleted, and a segment of the real PVR waveform with an initial interval is trimmed.

10. The system (10) for estimating CBPs using a PPG signal analysis according to claim 7, wherein the modeling-used PPG waveform signals and the real PVR waveforms are synchronized with each other using a same peak number alignment and dynamic time warping method.

**11.** The system (10) for estimating CBPs using a PPG signal analysis according to claim 7, wherein the upper-arm wearable apparatus (11) further includes a gravity sensor sensing a motion of the upper-arm of the subject when the subject wears the upper-arm wearable apparatus.

**12.** The system (10) for estimating CBPs using a PPG signal analysis according to claim 11, wherein the upper-arm wearable apparatus (11) further includes a reminder device which alerts the subject when the gravity sensor sensing the motion.

**13.** The system (10) for estimating CBPs using a PPG signal analysis according to claim 7, wherein the PPG signal receiver and analyzer (13), the PPG to BP estimator and calibrator (14), the PPG to PVR transformer (15) and the CBP estimator (16) are integrated into the upper-arm wearable apparatus (11).

**14.** The system (10) for estimating CBPs using a PPG signal analysis according to claim 7, wherein the upper-arm wearable apparatus (11) wirelessly or by wire transmits the PPG waveform signals to the PPG signal receiver and analyzer (13).

**15.** The system (10) for estimating CBPs using a PPG signal analysis according to claim 7, wherein the PPG signal receiver and analyzer (13) calibrates the estimated BPs through machine learning (ML) algorithms using an age and gender grouping method.

FIG. 1

FIG. 2A

FIG. 2B

FIG. 3

**Real SBP versus Estimated SBP**

FIG. 4A

**Real SBP versus Estimated SBP**

FIG. 4B

FIG. 5A

FIG. 5B

EP 4 245 213 A1

FIG. 5C

FIG. 5D

Input PPG Signal

FIG. 6A

Preprocessed PPG Signal

FIG. 6B

Approximate PVR Waveform

FIG. 6C

Refined PVR Waveform

FIG. 6D

FIG. 7A

FIG. 7B

FIG. 7C

FIG. 7D

EP 4 245 213 A1

**EP 4 245 213 A1**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | CHEN JIA-WEI ET AL: "A Data-Driven Model with Feedback Calibration Embedded Blood Pressure Estimator Using Reflective Photoplethysmography", SENSORS, vol. 22, no. 5, 27 February 2022 (2022-02-27), page 1873, XP93045205, DOI: 10.3390/s22051873 | 1-6 | INV. A61B5/022 A61B5/024 A61B5/00 |
| A | * abstract * <br> * page 2 – page 14 * <br> * figures 1A-C,4,6 * <br> * table 2 * <br> ----- | 7-15 | |
| A | WO 2018/167728 A1 (ATCOR MEDICAL PTY LTD [AU]) 20 September 2018 (2018-09-20) <br> * abstract * <br> * paragraph [0037] * <br> * paragraph [0047] – paragraph [0063] * <br> * figures 1A,5-8 * <br> ----- | 1-15 | |
| A | US 5 776 071 A (INUKAI HIDEKATSU [JP] ET AL) 7 July 1998 (1998-07-07) <br> * abstract * <br> * column 2, line 3 – line 45 * <br> * column 5, line 41 – column 10, line 3 * <br> * figures 1,2,5 * <br> ----- <br> -/-- | 1-15 | TECHNICAL FIELDS SEARCHED (IPC) <br><br> A61B |

The present search report has been drawn up for all claims

| Place of search | Date of completion of the search | Examiner |
|---|---|---|
| The Hague | 10 May 2023 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches Patentamt
European Patent Office
Office européen des brevets

# EUROPEAN SEARCH REPORT

Application Number

EP 22 19 2062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | NABIL IBTEHAZ ET AL: "PPG2ABP: Translating Photoplethysmogram (PPG) Signals to Arterial Blood Pressure (ABP) Waveforms using Fully Convolutional Neural Networks", ARXIV.ORG, CORNELL UNIVERSITY LIBRARY, 201 OLIN LIBRARY CORNELL UNIVERSITY ITHACA, NY 14853, 4 May 2020 (2020-05-04), XP091322443, * Sections "1.Introduction" and "2.Materials and Methods" * * figures 1-3 * | 7-15 | |
| A,D | US 2015/272512 A1 (CHEN CHEN-HUAN [TW] ET AL) 1 October 2015 (2015-10-01) * abstract * * paragraph [0003] * * paragraph [0010] - paragraph [0016] * * paragraph [0025] - paragraph [0039] * * figures 1-3 * | 1-15 | |
| | | | TECHNICAL FIELDS SEARCHED (IPC) |

The present search report has been drawn up for all claims

| Place of search | Date of completion of the search | Examiner |
|---|---|---|
| The Hague | 10 May 2023 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 245 213 A1

## ANNEX TO THE EUROPEAN SEARCH REPORT
## ON EUROPEAN PATENT APPLICATION NO.

EP 22 19 2062

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2023

| Patent document cited in search report | | | Publication date | Patent family member(s) | | | Publication date |
|---|---|---|---|---|---|---|---|
| WO 2018167728 | A1 | | 20-09-2018 | AU | 2018235369 | A1 | 31-10-2019 |
| | | | | CA | 3056887 | A1 | 20-09-2018 |
| | | | | CN | 110621219 | A | 27-12-2019 |
| | | | | JP | 7123070 | B2 | 22-08-2022 |
| | | | | JP | 2020510495 | A | 09-04-2020 |
| | | | | US | 2018263513 | A1 | 20-09-2018 |
| | | | | WO | 2018167728 | A1 | 20-09-2018 |
| US 5776071 | A | | 07-07-1998 | DE | 69715290 | T2 | 31-07-2003 |
| | | | | EP | 0804899 | A1 | 05-11-1997 |
| | | | | EP | 1195132 | A1 | 10-04-2002 |
| | | | | ES | 2183040 | T3 | 16-03-2003 |
| | | | | JP | 3666987 | B2 | 29-06-2005 |
| | | | | JP | H09294728 | A | 18-11-1997 |
| | | | | US | 5776071 | A | 07-07-1998 |
| US 2015272512 | A1 | | 01-10-2015 | CN | 103767693 | A | 07-05-2014 |
| | | | | TW | 201402067 | A | 16-01-2014 |
| | | | | US | 2015272512 | A1 | 01-10-2015 |
| | | | | WO | 2014063451 | A1 | 01-05-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63321095
- US 75291122

- US 201502725112 A

**Non-patent literature cited in the description**

- **JIA-WEI CHEN et al.** A Data-Driven Model with Feedback Calibration Embedded Blood Pressure Estimator Using Reflective Photoplethysmography. *Sensors,* 2022, vol. 22 (5), 1873

- **NABIL IBTEHAZ ; M. SOHEL RAHMAN.** PPG2ABP: Translating Photoplethysmogram (PPG) Signals to Arterial Blood Pressure (ABP) Waveforms using Fully Convolutional Neural Networks. *Electrical Engineering and Systems Science: Signal Processing,* 05 May 2020